# EUROPÄISCHE PATENTSCHRIFT

(11) **EP 0 581 003 B1**
(45) Veröffentlichungstag und Bekanntmachung des Hinweises auf die Patenterteilung: **06.09.2000**
(21) Anmeldenummer: 93109465.0
(22) Anmeldetag: 14.06.1993
(51) Int. Cl.: C07D 233/68, C07D 403/10, A61K 31/415, C07C 69/75

(54) **Imidazolyl-substituierte Cyclohexanderivate**
Imidazolyl substituted cyclohexane derivatives
Dérivés du cyclohexane substitués par imidazole

(30) Priorität: 26.06.1992 DE 4221009
(43) Veröffentlichungstag der Anmeldung: 02.02.1994
(73) Patentinhaber: BAYER AG, 51368 Leverkusen (DE)
(72) Erfinder: Müller, Ulrich E., Dr., D-5600 Wuppertal (DE); Dressel, Jürgen, Ph.D., D-5600 Wuppertal (DE); Fey, Peter, Dr., D-5600 Wuppertal (DE); Hanko, Rudolf, Dr., D-4000 Düsseldorf (DE); Hübsch, Walter, Dr., D-5600 Wuppertal (DE); Krämer, Thomas, Dr., D-5600 Wuppertal (DE); Müller-Gliemann, Matthias, Dr., D-5650 Solingen-Ohligs (DE); Beuck, Martin, Dr., D-4006 Erkrath 2 (DE); Kazda, Stanislav, Prof. Dr., D-5600 Wuppertal (DE); Knorr, Andreas, Dr., D-4006 Erkrath 2 (DE); Stasch, Johannes-Peter, Dr., D-5600 Wuppertal (DE); Wohlfeil, Stefan, Dr., D-4010 Hilden (DE)

(56) Entgegenhaltungen:
- EP-A- 0 253 310
- EP-A- 0 324 377

## Beschreibung

Die Erfindung betrifft Imidazolyl-substituierte Cyclohexanderivate, Verfahren zu ihrer Herstellung sowie ihre Verwendung in Arzneimitteln, insbesondere als blutdrucksenkende und antiatherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigerndes Oktapeptid Angiotensin II abgebaut wird. Die verschiedenen Effekte des Angiotensin II, wie beispielsweise Vasokonstriktion, Na⁺-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Außerdem sind Imidazolderivate mit Angiotensin II inhibierender Wirkung aus WO 91/ 00-281-A, WO 91 / 00-277-A, der EP 253 303 A, EP 253 310 und EP 324 377 bekannt.

In EP-A-0 324 377 und in EP-A-0 253 310 werden unter anderem Imidazolylbenzylsubstituierte Cyclohexylcarbonsäuren mit Angiotensin II antagonistischer Wirkung beschrieben. Die Verbindungen haben aufgrund dessen antihypertensive Eigenschaften und können daher als Wirkstoffe in Arzneimitteln zur Behandlung von Bluthochdruck eingesetzt werden.

Die vorliegende Erfindung betrifft Imidazolyl-substituierte Cyclohexanderivate der allgemeinen Formel (I) 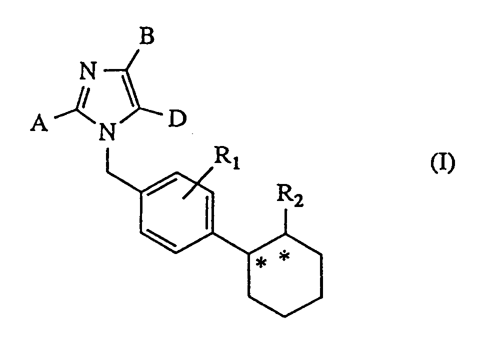 in welcher
- A: für geradkettiges oder verzweigtes Alkyl oder Alkenyl mit jeweils bis zu 8 Kohlenstoffatomen steht, oder für Cycloalkyl mit 3 bis 8 Kohlenstoffatomen steht,
- B: für Wasserstoff, Halogen oder für Perfluoralkyl mit bis zu 5 Kohlenstoffatomen steht,
- D: für eine Gruppe der Formel-CH₂-OR³ oder -CO-R⁴ steht,
worin
- R³: Wasserstoff oder geradkettiges oder verzweigtes Alkyl mit bis zu 8 Kohlenstoffatomen bedeutet,
- R⁴: Wasserstoff, Hydroxy oder geradkettiges oder verzweigtes Alkoxy mit bis zu 8 Kohlenstoffatomen bedeutet,
- R¹: für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano oder Carboxy steht,
- R²: für einen Rest der Formel -CO-NR⁶R⁷ steht,
worin
- R⁶: Wasserstoff oder geradkettiges oder verzweigtes Alkyl mit bis zu 6 Kohlenstoffatomen bedeutet,
- R⁷: einen Rest der Formel -SO₂R⁹ oder 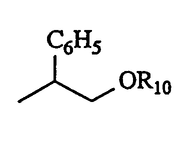 bedeutet,
worin
- R⁹: geradkettiges oder verzweigtes Alkyl mit bis zu 8 Kohlenstoffatomen bedeutet, das gegebenenfalls durch Phenyl oder Tolyl substituiert ist, oder Phenyl bedeutet, das gegebenenfalls durch Halogen oder durch geradkettiges oder verzweigtes Alkyl mit bis zu 6 Kohlenstoffatomen substituiert ist,
- R¹⁰: Wasserstoff, geradkettiges oder verzweigtes Alkyl mit bis zu 6 Kohlenstoffatomen oder eine Hydroxyschutzgruppe bedeutet,
- R⁸: Wasserstoff, geradkettiges oder verzweigtes Alkyl mit bis zu 4 Kohlenstoffatomen oder die Triphenylmethylgruppe bedeutet
und deren Salze.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der Imidazolyl-substituierten Cyclohexanderivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Hydroxyschutzgruppe im Rahmen der oben angegebenen Definition steht im allgemeinen für eine Schutzgruppe aus der Reihe: Trimethylsilyl, Triethylsilyl, Triisopropylsilyl, tert.Butyl-dimethylsilyl, tert.-Butyldiphenylsilyl, Trimethylsilylethoxycarbonyl, Benzyl, Triphenylmethyl(Trityl), Monomethoxytrityl (MMTr), Dimethoxytritryl (DMTr), Benzyloxycarbonyl, 2-Nitrobenzyl, 4-Nitrobenzyl, 2-Nitrobenzyloxycarbonyl, 4-Nitrobenzyloxycarbonyl, tert. Butyloxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methoxymethyl, Methylthiomethyl, Methoxyethoxymethyl, [2-(Trimethylsilyl)ethoxy]-methyl, 2-(Methylthiomethoxy)ethoxycarbonyl, Tetrahydropyranyl, Benzoyl, 4-Methylbenzoyl, 4-Nitrobenzoyl, 4-Fluorbenzoyl, 4-Chlorbenzoyl oder 4-Methoxybenzoyl. Bevorzugt ist Acetyl.

Bevorzugt sind Verbindungen der allgemeinen Formel (I),
in welcher
- A: für geradkettiges oder verzweigtes Alkyl oder Alkenyl mit jeweils bis zu 6 Kohlenstoffatomen steht, oder für Cyclopropyl, Cyclobutyl, Cyclopentyl, Cyclohexyl oder Cycloheptyl steht,
- B: für Wasserstoff, Fluor, Chlor, Brom oder für Perfluoralkyl mit bis zu 4 Kohlenstoffatomen steht,
- D: für eine Gruppe der Formel -CH₂OR³ oder -CO-R⁴ steht,
worin
- R³: Wasserstoff oder geradkettiges oder verzweigtes Alkyl mit bis zu 6 Kohlenstoffatomen bedeutet,
- R⁴: Wasserstoff, Hydroxy oder geradkettiges oder verzweigtes Alkoxy mit bis zu 6 Kohlenstoffatomen bedeutet,
- R¹: für Wasserstoff, Fluor, Chlor, Brom, Trifluormethyl, Carboxy oder geradkettiges oder verzweigtes Alkyl, Alkoxy oder Alkoxycarbonyl mit jeweils bis zu 4 Kohlenstoffatomen steht,
- R²: für einen Rest der Formel CO-NR⁶R⁷ steht,
worin
- R⁶: Wasserstoff oder geradKettiges oder verzweigtes Alkyl mit bis zu 4 Kohlenstoffatomen bedeutet,
- R⁷: einen Rest der Formel -SO₂-R⁹ oder 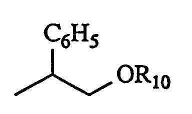 bedeutet, worin
- R⁹: geradkettiges oder verzweigtes Alkyl mit bis zu 6 Kohlenstoffatomen bedeutet, das gegebenenfalls durch Phenyl oder Tolyl substituiert ist, oder Phenyl bedeutet, das gegebenenfalls durch Fluor, Chlor, Brom, oder durch geradkettiges oder verzweigtes Alkyl mit bis zu 4 Kohlenstoffatomen substituiert ist,
- R¹⁰: Wasserstoff oder geradkettiges oder verzweigtes Alkyl mit bis zu 4 Kohlenstoffaotmen oder Benzyl bedeutet,
- R⁸: Wasserstoff, Methyl, Ethyl oder die Triphenylmethylgruppe bedeutet,
und deren Salze.

Besonders bevorzugt sind Verbindungen der allgemeinen Formel (I),
in welcher
- A: für geradkettiges oder verzweigtes Alkyl oder Alkenyl mit jeweils bis zu 4 Kohlenstoffatomen steht, oder für Cyclopropyl, Cyclopentyl oder Cyclohexyl steht,
- B: für Wasserstoff, Fluor, Chlor oder für Perfluoralkyl mit bis zu 3 Kohlenstoffatomen steht,
- D: für eine Gruppe der Formel -CH₂OR³ oder -CO-R⁴ steht,
worin
- R³: Wasserstoff oder geradkettiges oder verzweigtes Alkyl mit bis zu 4 Kohlenstoffatomen bedeutet,
- R⁴: Wasserstoff, Hydroxy oder geradkettiges oder verzweigtes Alkoxy mit bis zu 4 Kohlenstoffatomen bedeutet,
- R¹: für Wasserstoff, Fluor, Chlor, Brom, Trifluormethyl oder Methyl steht,
- R²: für einen Rest der Formel -CO-NR⁶R⁷ steht,
worin
- R⁶: Wasserstoff, Methyl oder Ethyl bedeutet,
- R⁷: einen Rest der Formel -SO₂R⁹ oder 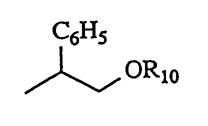 bedeutet,
worin
- R⁹: geradkettiges oder verzweigtes Alkyl mit bis zu 4 Kohlenstoffatomen bedeutet, das gegebenenfalls durch Phenyl oder Tolyl substituiert ist, oder Phenyl oder Tolyl bedeutet,
- R¹⁰: Wasserstoff, Methyl oder Ethyl bedeutet,
- R⁸: Wasserstoff, Methyl oder die Triphenylmethylgruppe bedeutet
und deren Salze.

Außerdem wurde ein Verfahren zur Herstellung der erfindungsgemäßen Verbindungen der allgemeinen Formel (I) gefunden, dadurch gekennzeichnet, daß man Cyclohexan-Verbindungen der allgemeinen Formel (II) 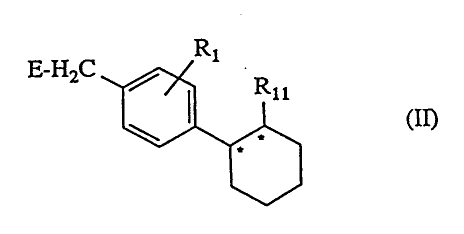 in welcher
- E: für Chlor, Brom, Jod, Tosylat oder Mesylat, vorzugsweise für Brom, steht,
- R¹: die oben angegebene Bedeutung hat
und
- R¹¹: geradkettiges oder verzweigtes C₁-C₄-Alkoxycarbonyl bedeutet,
zunächst mit Imidazolen der allgemeinen Formel (III) 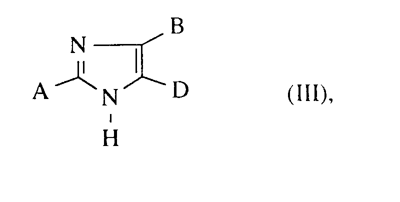 in welcher
- A, B und D: die oben angegebene Bedeutung haben,
in inerten Lösemitteln, gegebenenfalls in Anwesenheit einer Base und gegebenenfalls unter Schutzgasatmosphäre zu Verbindungen der allgemeinen Formel (IV) 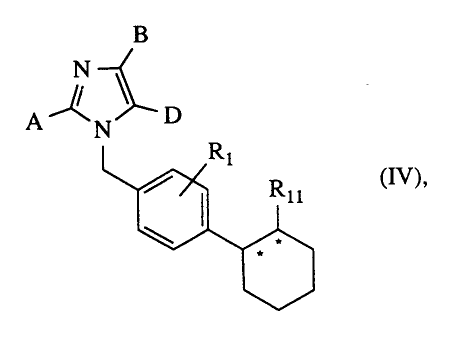 in welcher
- A, B, D, R¹ und R¹¹: die oben angegebene Bedeutung haben,
umsetzt,
im Fall der Säuren (R²=CO₂H) die Ester verseift,
und im Fall der Amide und Sulfonamide, ausgehend von den entsprechenden Carbonsäuren, nach vorgeschalteter Aktivierung mit Verbindungen der allgemeinen Formel (V)

H-NR⁶R⁷ (V)

in welcher
- R⁶ und R⁷: die oben angegebene Bedeutung haben,
gegebenenfalls in Anwesenheit einer Base und/oder eines Hilfsstoffes, beispielsweise eines Dehydratisierungsmittels, in inerten Lösemitteln amidiert,
und gegebenenfalls die Substituenten A, B, D und R¹ nach üblichen Methoden, beispielsweise durch Reduktion, Oxidation, Alkylierung oder Hydrolyse einführt oder in andere Gruppen überführt,
und gegebenenfalls die Isomeren trennt, und im Fall der Herstellung der Salze mit einer entsprechenden Base oder Säure umsetzt.

Das erfindungsgemäße Verfahren kann durch folgendes Formelschema beispielhaft erläutert werden:

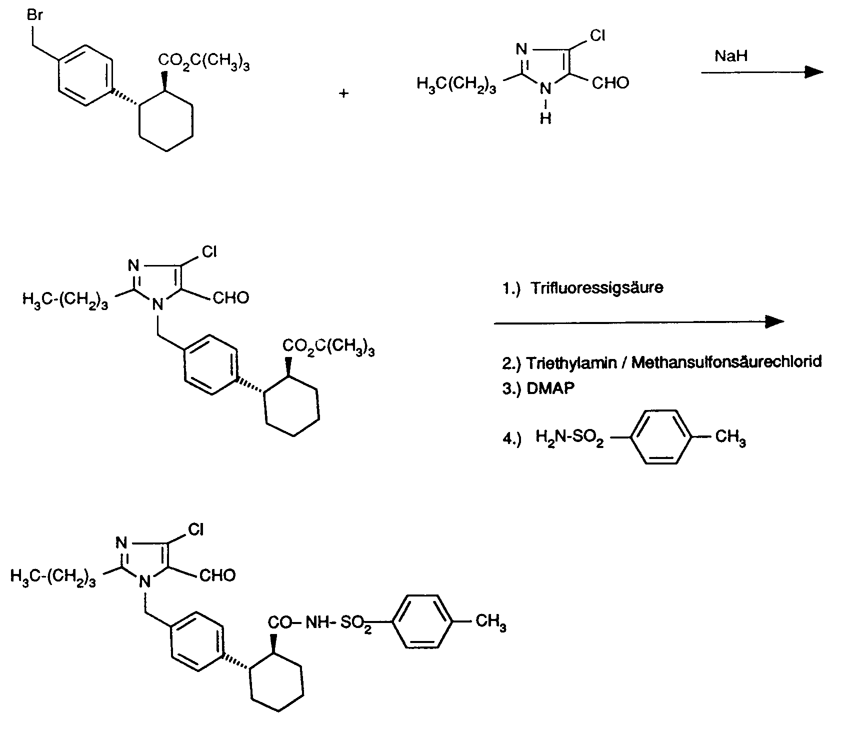

Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dimethylformamid und Tetrahydrofuran.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali-oder Erdalkalialkoholate wie Natrium- oder Kaliummethanolat, Natrium- oder Kaliumethanolat oder Kalium-tert.butylat, oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle wie Natrium oder deren Hydride wie Natriumhydrid einzusetzen. Bevorzugt sind Natriumhydrid, Kaliumcarbonat, Triethylamin, Pyridin und Kalium-tert.butylat.

Im allgemeinen setzt man die Base in einer Menge von 0,05 mol bis 10 mol, bevorzugt von 1 mol bis 2 mol bezogen auf 1 mol der Verbindung der Formel (III) ein.

Das erfindungsgemäße Verfahren wird im allgemeinen in einem Temperaturbereich von -30°C bis +100°C, bevorzugt von -10°C bis +60°C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Basen eignen sich für die Hydrolyse die üblichen anorganischen Basen. Hierzu gehören bevorzugt Alkalihydroxide oder Erdalkalihydroxide wie beispielsweise Lithiumhydroxid, Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Lithiumhydroxid, Natriumhydroxid oder Kaliumhydroxid eingesetzt.

Als Lösemittel eignen sich für die Hydrolyse Wasser oder die für eine Hydrolyse üblichen organischen Lösemittel. Hierzu gehören bevorzugt Alkohole wie Methanol, Ethanol, Propanol, Isopropanol, Butanol oder tert.Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Bevorzugt erfolgt die Hydrolyse mit Säuren wie beispielsweise Trifluoressigsäure, Essigsäure, Chlorwasserstoffsäure, Chlorwasserstoffsäure Dioxan, Bromwasserstoffsäure, Methansulfonsäure, Schwefelsäure oder Perchlorsäure. Besonders bevorzugt sind Trifluoressigsäure oder Chlorwasserstoffsäure in Dioxan.

Die Hydrolyse wird im allgemeinen in einem Temperaturbereich von 0°C bis +100°C, bevorzugt von +20°C bis +80°C, durchgeführt.

Im allgemeinen wird die Hydrolyse bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol, bezogen auf 1mol des Esters, eingesetzt. Besonders bevorzugt verwendet man molare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Säuren wie beispielsweise Chlorwasserstoffsäure, Bromwasserstoffsäure, Schwefelsäure, Phosphorsäure oder Trifluoressigsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern. Die Säuren können dann in üblicher Weise isoliert werden.

Die Amidierung und die Sulfoamidierung ausgehend von den Verbindungen der allgemeinen Formel (IV), erfolgen im allgemeinen in einem der oben aufgeführten Lösemittel, vorzugsweise in Tetrahydrofuran oder Dichlormethan.

Die Amidierung und die Sulfoamidierung können gegebenenfalls über die aktivierte Stufe der Säurehalogenide oder gemischter Anhydride, die aus den entsprechenden Säuren durch Umsetzung mit Thionylchlorid, Phosphortrichlorid, Phosphorpentachlorid, Phosphortribromid, Oxalylchlorid oder Methansulfonsäurechlorid hergestellt werden können, verlaufen.

Die Amidierung und die Sulfoamidierung erfolgen im allgemeinen in einem Temperaturbereich von -50°C bis +80°C, vorzugsweise von -30°C bis +20°C, und Normaldruck.

Als Basen eignen sich dafür neben den oben aufgeführten Basen vorzugsweise Triethylamin und/oder (Dimethylamino)pyridin, DBU oder DABCO.

Die Base wird in einer Menge von 0,5 mol bis 10 mol, bevorzugt von 1 mol bis 5 mol, bezogen auf 1 mol der Verbindungen der allgemeinen Formel (IV), eingesetzt.

Als säurebindende Mittel für die Amidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo-[3.4.0]-nonene-5 (DBN) oder 1,5-Diazabicyclo[3.4.0]undecene-5 (DBU) eingesetzt werden. Bevorzugt ist Triethylamin.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexylfluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid [vgl. J.C. Sheehan, S.L. LEdis, J. Am. Chem. Soc. 95, 875 (1973); F.E. Frerman et al., J. Biol. Chem. 225, 507 (1982) und N.B. Benoton, K. Kluroda, Int. Pept. Prot. Res. 13, 403 (1979), 17, 187 (1981)].

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die oben aufgeführte Derivatisierung der Substituenten A, B, D und R¹ erfolgt im allgemeinen nach literaturbekannten Methoden, wobei beispielhaft die Reduktion von Aldehyden oder Alkoxycarbonylverbindungen zu Alkoholen (a), die Oxidation von Aldehyden zu Carbonsäuren, (b) und die Alkylierung (c) mit folgendem erläutert werden sollen:
a) Die Reduktion von Alkoxycarbonylverbindungen oder Aldehyden zu den entsprechenden Alkoholen erfolgt im allgemeinen mit Hydriden, wie Natriumborhydrid oder Natriumborhydrid, bevorzugt mit Lithiumaluminiumhydrid in inerten Lösemitteln wie Ethern, Kohlenwasserstoffen oder Alkoholen oder deren Gemischen, bevorzugt in Ethern wie beispielsweise Diethylether, Tetrahydrofuran oder Dioxan, oder Alkoholen wie Ethanol, im Fall der Aldehyde bevorzugt mit Natriumborhydrid in Ethanol, in einem Temperaturbereich von 0°C bis +150°C, bevorzugt von +20°C bis +100°C, bei Normaldruck.
b) Die Oxidation von Aldehyden zu den Carbonsäuren erfolgt im allgemeinen in einem der oben aufgeführten Lösemitteln, vorzugsweise tert.Butanol, mit Kaliumpermanganat, in Anwesenheit von Natriumhydrogenphosphat und Natriumsulfit, in einem Temperaturbereich von -30°C bis +20°C, bevorzugt von -20°C bis +20°C und Normaldruck.
C) Die Alkylierung erfolgt im allgemeinen in einem der oben aufgeführten Lösemitteln mit Alkylierungsmitteln wie beispielsweise (C₁-C₈)-Alkylhalogenide, Sulfonsäureester oder substituierte oder unsubstituierte (C₁-C₆)-Dialkyl- oder (C₁-C₁₀)-Diarylsulfate, vorzugsweise Methyljodid, p-Toluolsulfonsäureester oder Dimethylsulfat.

Die Cyclohexan-Verbindungen der allgemeinen Formel (II) sind neu und können hergestellt werden, indem man Verbindungen der allgemeinen Formel (VI) 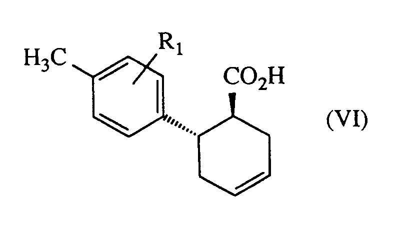 in welcher
- R¹: die oben angegebene Bedeutung hat,
zunächst durch Hydrierung mit Palladium/C in einem der oben angegebenen Lösemitteln, vorzugsweise Methanol, in einer Wasserstoffatmosphäre in die Verbindungen der allgemeinen Formel (VII) 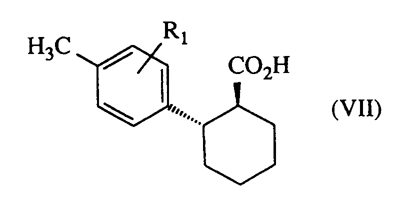 in welcher
- R¹: die oben angegebene Bedeutung hat,
überführt,
in einem zweiten Schritt, nach üblichen Methoden verestert.

Die Reduktion der Doppelbindung erfolgt in einem Temperaturbereich von 0°C bis +40°C, vorzugsweise bei +20°C und einem Druck von 1 bar.

Die Veresterung erfolgt in einem der oben angegebenen Lösemitteln, vorzugsweise Toluol und Tetrahydrofuran, nach der oben bereits beschriebenen vorgeschalteten Aktivierung der entsprechenden Carbonäsure, vorzugsweise über die Carbonsäurechloride, und nachträglicher Umsetzung mit dem entsprechenden Alkoholaten, in einem Temperaturbereich von 0°C bis +60°C, vorzugsweise bei +10°C bis +35°C und Normaldruck.

Die Bromierung wird im allgemeinen in einem Temperaturbereich von +40°C bis 100°C, vorzugsweise von +60°C bis +90°C und Normaldruck durchgeführt. Sie erfolgt in einem der oben angegebenen Lösemitteln, vorzugsweise mit Tetrachlorkohlenstoff, und mit N-Bromsuccinimid.

Als Starter (Katalysator) für die Bromierung eignen sich beispielsweise Azobisisobutyronitril, Dibenzoylperoxid, vorzugsweise Azobisisobutyronitril, wobei der Starter in einer Menge von 0,01 mol bis 0,1 mol, bevorzugt von 0,01 mol bis 0,05 mol, bezogen auf 1 mol der Verbindung der allgemeinen Formel (VII), eingesetzt wird.

Die Verbindungen der allgemeinen Formel (VI) sind ebenfalls neu und können beispielsweise hergestellt werden, indem man Verbindungen der allgemeinen Formel (VIII) 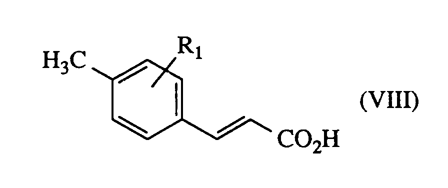 in welcher
- R¹: die oben angegebene Bedeutung hat,
in einem der oben angegebenen Lösemittel, vorzugsweise Toluol, mit 1,3-Butadien in Anwesenheit von Hydrochinon, in einem Temperaturbereich von +180°C bis +230°C, vorzugsweise bei 200°C und einem Druck von ca. 20 bar umsetzt [vgl. hierzu Eur. J. Med. Chem. 11, 493 (1976)].

Die Verbindungen der allgemeinen Formel (VIII) sind an sich bekannt oder können nach üblichen Methoden hergestellt werden [vgl. Organikum, VEB Deutscher Verlag der Wissenschaften, Berlin 1977, Seiten 572 ff.].

Die Verbindungen der allgemeinen Formeln (IV) und (VII) sind neu und können beispielsweise nach den oben beschriebenen Verfahren hergestellt werden.

Ebenso sind die Verbindungen der allgemeinen Formel (III) an sich bekannt [vgl. z.B. EP 324 377, Beilstein 25, 163; 23, 45; US 4 355 040] oder können nach üblicher Methode hergestellt werden.

Die Amine der allgemeinen Formel (V) sind bekannt oder können nach bekannten Verfahren hergestellt werden [vgl. z.B. Beilstein 11/104, R.V. Vitzgert, Uspekhi, Khimii 32, 3 (1963); Russian Chem Rev 32, 1 (1969); Beilstein 4, 87].

Die erfindungsgemäßen Verbindungen der allgemeinen Formel (I) zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretions-stimulierenden Effekte des Angiotensin II. Darüber hinaus inhibieren sie die Proliferation von glatten Muskelzellen.

Sie können deshalb in Arzneimitteln zur Behandlung der arteriellen Hypertonie und Atherosklerose eingesetzt werden. Darüber hinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

### Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogen-begaster Krebs-Henseleit-Nährlösung folgender Zusammensetzung: 119 mmol/l NaCl; 2,5 mmol/l CaCl₂ x 2 H₂O; 1,2 mmol/l KH₂PO₄; 10 mmol/l Glucose; 4,8 mmol/l KCl; 1,4 mmol/l MgSO₄ x7 H₂O und 25 mmol/l NaHCO₃ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

| Agonisten und ihre Standardkonzentrationen (Applikationsvolumen pro Einzelgabe = 100 µl): | | |
|---|---|---|
| KCl | 22,7;32,7;42,7;52,7 | mmol/l |
| 1Noradrenalin | 3x10⁻⁹;3x10⁻⁸;3x10⁻⁷;3x10⁻⁶ | g/ml |
| Serotonin | 10⁻⁸;10⁻⁷;10⁻⁶;10⁻⁵ | g/ml |
| B-HT 920 | 10⁻⁷;10⁻⁶;10⁻⁵ | g/ml |
| Methoxamin | 10⁻⁷;10⁻⁶;10⁻⁵ | g/ml |
| Angiotensin II | 3x10⁻⁹;10⁻⁸;3x10⁻⁸;10⁻⁷ | g/ml |

Für die Berechnung der IC₅₀ (Konzentration, bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

**Tabelle A**

| Hemmung der Gefäßkontraktion an isolierten Aortenringen von Kaninchen in vitro | |
|---|---|
| IC₅₀ (g/ml) gegen Kontraktionen, induziert durch AII | |

| Bsp.Nr.: | IC₅₀ [nM] |
|---|---|
| 4 | 1400 |
| 9 | 920 |
| 13 | 240 |

### Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

### Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.
Der arterielle Blutdruck dieser Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

### Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.
Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), ³H-Angiotensin II (3-5 nM), Test-Pufferlösung (50 mM Tris, pH 7,2, 5 mM MgCl₂ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu Kᵢ- bzw. IC₅₀-Werten (Kᵢ: für die verwendete Radioaktivität korrigierte IC₅₀-Werte; IC₅₀-Werte: Konzentration bei der die zu untersuchende Substanz eine 50%ige Hemmung der spezifischen Bindung des Radioliganden bewirkt).

**Tabelle B**

| Bsp.-Nr. | Ki [nM] |
|---|---|
| 9 | 120 |
| 13 | 72 |

### Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Schweinen durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5 % FCS und 7,5 % NCS 2 mM L-Glutamin und 15 mmol HEPES, pH 7,4 in 5% CO₂ bei 37°C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird 1 µCi ³H-Thymidin zugefügt und nach weiteren 4 Stunden der Einbau dieser Substanz in die TCA-präzipitierbare DNA der Zellen bestimmt.

Zur Bestimmung der IC₅₀-Werte wird die Wirkstoffkonzentration errechnet, die bei sequentieller Verdünnung des Wirkstoffes halbmaximale Hemmung der durch 1 % FCS hervorgerufenen Thymidin in Korporation bewirkt.

**Tabelle C**

| Bsp.-Nr. | Inhibition [%] bei 10⁻⁶M |
|---|---|
| 6 | 41 |
| 7 | 100 |
| 10 | 12 |
| 11 | 100 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### DC-Laufmittelgemische

- A: = Dichlormethan : Methanol = 5:1
- B: = Dichlormethan : Methanol = 3:1
- C: = Dichlormethan : Methanol = 20:1
- D: = Dichlormethan : Methanol = 10:1
- E: = Petrolether : Essigester = 10:1
- F: = Petrolether : Essigester = 5:1
- G: = Dichlormethan : Methanol = 100:1
- H: = Petrolether : Essigester = 1:1

### Ausgangsverbindungen

### Beispiel I

### trans-6-(4-Tolyl)-cyclohex-3-en-1-carbonsäure

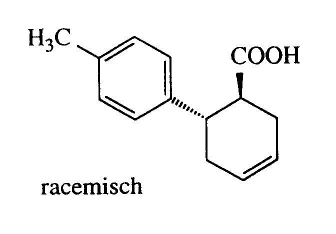

275 g (1,695 mol) 3-(4-Toluol)acrylsäure (käuflich bei Aldrich) wird nach einem bekannten Verfahren [vgl.: Eur. J. Med. Chem. 11, 493 (1976)] in 480 ml Toluol mit 580 ml 1,3-Butadien (in kondensierter Form abgemessen) unter Zugabe von 3 g Hydrochinon 22 h bei ca. 200°C und ca. 20 bar umgesetzt Das Rohgemisch wird mit Toluol verdünnt und mit 0,5 M wäßriger Natronlauge extrahiert. Darauf werden die wäßrigen Phasen mit 1 M Salzsäure angesäuert und mit Ether extrahiert Die etherischen Lösungen werden mit Natriumsulfat getrocknet, eingedampft und wiederum in Toluol gelöst. Nach 15 minütigem Kochen mit 5 g Aktivkohle saugt man heiß ab und dampft das Lösemittel bis auf ca. 120 - 160 ml ab; bei 0 - 4°C kristallisieren 124 g (573 mol) Produkt aus. Das Filtrat wird etwas weiter eingeengt und zum Nachkristallisieren wieder abgekühlt. Bei Wiederholung dieses Vorgangs fallen insgesamt weitere 42 g (194 mmol) Produkt an.
R_{f} = 0,39 (D)

### Beispiel II

### trans-2-(4-Tolyl)-cyclohexan-1-carbonsäure

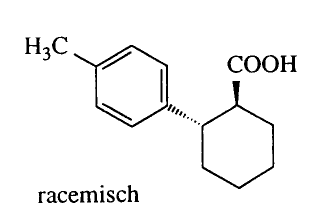

155 g (717 mmmol) der Verbindung aus Beispiel I werden in 1 l Methanol gelöst und an 10 g Palladium (10% auf Tierkohle) bei 20°C und Wasserstoffatmosphäre von ca. 1 bar umgesetzt. Nach einer Reaktionszeit von insgesamt 16 h wird der Katalysator abfiltriert und das Lösemittel - zuletzt im Hochvakuum - abgedampft.
Ausbeute: 153 g (701 mmol)
R_{f} = 0,38 (D)

### Beispiel III (Methode A)

### trans-2-(4-Tolyl)-cyclohexan-1-carbonsäure-tert.butylester

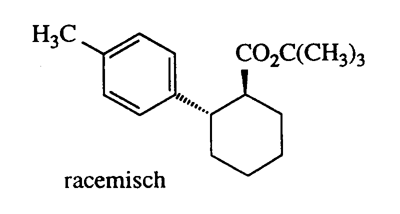

45,8 g (184 mmol) der Verbindung aus Beispiel II werden in 600 ml Toluol gelöst und unter Rückfluß mit 49,5 ml (387 mmol) Oxalylchlorid zur Reaktion gebracht. Nach 2 h dampft man das Lösemittel mit überschüssigem Reagenz ab; dazu muß das rohe Carbonsäurechlorid gegebenenfalls wiederholt in Toluol aufgenommen und noch einmal abrotiert werden. Das so gewonnene Produkt wird in 500 ml Tetrahydrofuran gelöst, mit 24,8 g (221 mmol) Kalium-tert.butanolat bei 0°C verrührt und 20 h nachgerührt (bei 20°C). Darauf werden Wasser und Ether zugesetzt und mehrfach extrahiert. Die organische Phase wird mit Natriumsulfat getrocknet, eingedampft und der Rückstand an Kieselgel 60 (Merck, Laufmittelgemisch C) chromatographisch gereinigt.
Ausbeute: 39,6 g (130 mmol)
R_{f} = 0,47 (E)

### (Methode B)

20,0 g (91,6 mmol) der Verbindung aus Beispiel II werden mit 7 ml konzentrierter Schwefelsäure in 100 ml Ether suspendiert und bei -30°C mit 80 ml (713 mmol) Isobuten versetzt (Druckapparatur). Das Gemisch wird in dem verschlossenen Gefäß auf 20°C erwärmt und über 20 Stunden umgesetzt. Darauf kühlt man wieder auf -30°C ab, öffnet die Apparatur und rührt das Reaktionsgemisch bei 20°C in 300 ml 3 M Natronlauge/400 ml Ether ein. Die wäßrige Phase wird mit Ether nachextrahiert, die organische Lösung mit Natriumsulfat getrocknet und eingedampft;
Ausbeute: 23,3 g (84,9 mmol).

### Beispiel IV

### trans-2-(4-Brommethylphenyl)-cyclohexan-1-carbonsäure-tert.butylester

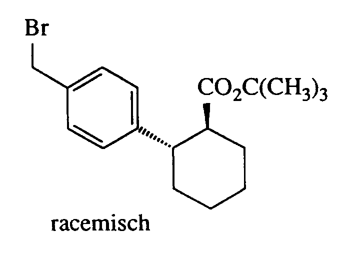

11,70 g (42,6 mmol) der Verbindung aus Beispiel III werden unter Rückfluß in 100 ml Tetrachlormethan mit 7,59 g (42,6 mmol) N-Bromsuccinimid und 1,4 g Azobisisobutyronitril umgesetzt. Nach einer Reaktionszeit von 4 h wird die Mischung abgekühlt, der angefallene Succinimidniederschlag abgesaugt und das Filtrat eingedampft.
Ausbeute: 14,2 g (40,2 mmol)
R_{f} = 0,48 (E)

### Herstellungsbeispiele

### Beispiel 1

### trans-2-[4-(2-Butyl-4-chlor-5-formyl-imidazol-1-yl-methyl)phenyl]cyclohexan-1-carbonsäure-tert.butylester

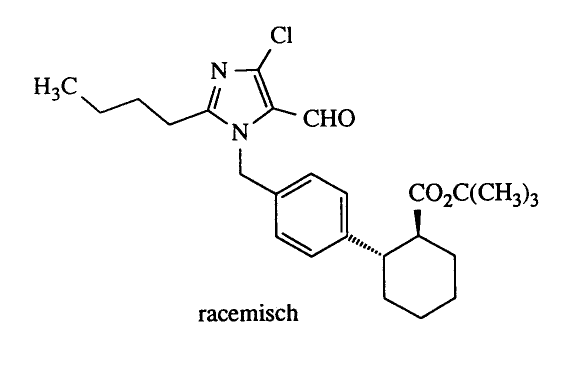

8,16 g (43,7 mmol) 2-Butyl-4-chlor-5-formyl-imidazol [EP 324 377] werden in 10 ml Dimethylformamid mit 1,32 g (43,7 mmol) Natriumhydrid (80%ig, stabilisiert mit Paraffin) bis zur Beendigung der Wasserstoffentwicklung bei ca. 0°C gerührt. Darauf tropft man eine Losung von 18,4 g (43,7 mmol) der Verbindung aus Beispiel IV in 100 ml Dimethylformamid zu und rührt 20 h bei 20°C nach. Zur Aufarbeitung gibt man Wasser zu und extrahiert mit Ether. Diese organischen Phasen werden mit Natriumsulfat getrocknet und einrotiert. Der erhaltene Rückstand wird chromatographisch an Kieselgel 60 (Merck, Laufmittel E) gereinigt.
Ausbeute: 7,81 g (17,0 mmol)
R_{f} = 0,67 (F)

### Beispiel 2

### trans-2-[4-(2-Butyl-4-chlor-5-formyl-imidazol-1-yl-methyl)phenyl]cyclohexan-1-carbonsäure

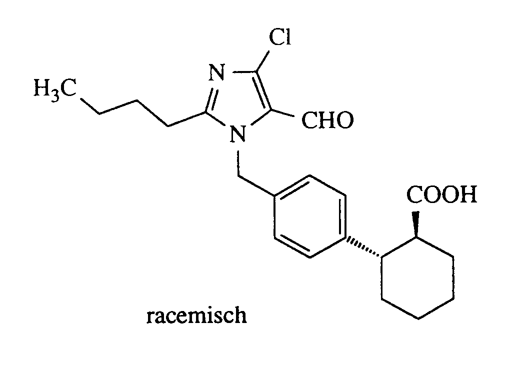

2,7 g (5,2 mmol) der Verbindung aus Beispiel 1 werden in 40 ml Dioxan mit 16 ml konzentrierter Salzsäure zur Reaktion gebracht Nach 18 h bei 20°C verdünnt man mit Ether, setzt 1 M wäßrige Natronlauge zu und schüttelt aus. Die wäßrige alkalische Phase (pH = 13 - 14) wird im Vakuum vom organischen Restlösemittel befreit und bei 0°C mit 2 M Salzsäure auf pH = 2 gestellt. Der ausfallende Niederschlag wird abgesaugt, mit Wasser gewaschen und im Hochvakuum über Natriumhydroxid und Phosphorpentoxid getrocknet.
Ausbeute: 2,0g (5,0 mmol)
R_{f} = 0,28 (C)

In Analogie zur Vorschrift des Beispiels 2 werden die in Tabelle 1 aufgeführten Verbindungen hergestellt:

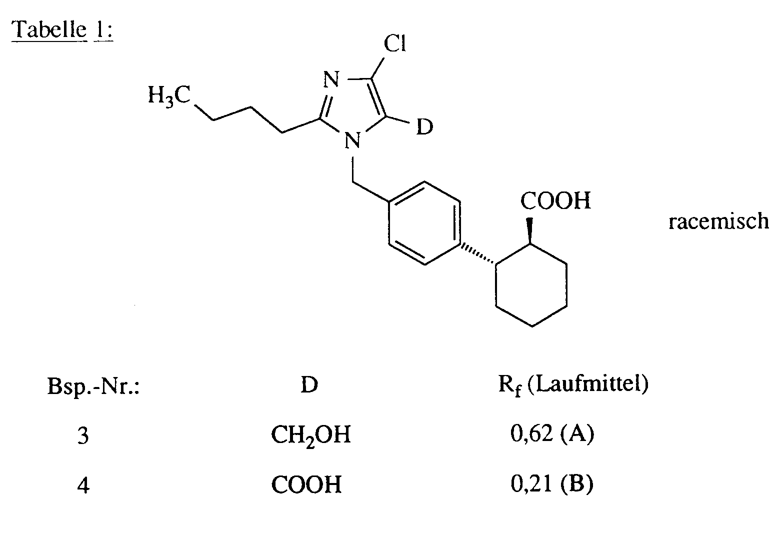

### Beispiel 5

### trans-2-[4-(2-Butyl-4-chlor-5-formyl-imidazol-1-yl-methyl)phenyl]cyclohexan-1-carbonsäure-N-(4-tolylsulfonyl)amid

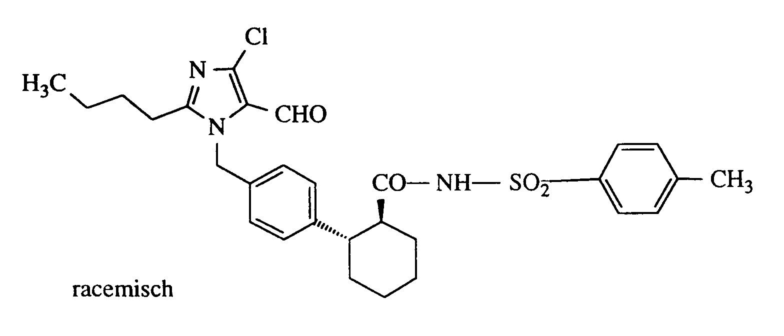

0,7 g (1,5 mmol) der Verbindung aus Beispiel 2 werden in 30 ml Tetrahydrofuran bei -20°C mit 0,127 ml (1,65 mmol) Methansulfonsäurechlorid und 0,91 ml (6,6 mmol) Triethylamin umgesetzt; nach 2 h bei dieser Temperatur werden 0,73 g (6,0 mmol) 4-(N,N-Dimethylamino)pyridin und 0,31 g (1,8 mmol) 4-Toluolsulfonsäureamid zugesetzt und 24 h bei 20°C nachgerührt. Die Reaktionsmischung wird dann auf 1 M Salzsäure gegossen und mehrfach mit Ether extrahiert. Die organischen Phasen werde mit Natriumsulfat getrocknet, eingedampft und der erhaltene Rückstand an Kieselgel 60 (Merck; Laufmittel G) chromatographisch gereinigt.
Ausbeute: 0,72 g (1,3 mmol)
R_{f} = 0,72 (C)

### Beispiel 6 und Beispiel 7

### [1,2-trans]-2-[4-(2-Butyl-4-chlor-5-formyl-imidazol-1-yl-methyl)phenyl]-cyclohexan-1-carbonsäure-(S)-phenylglycinolamid

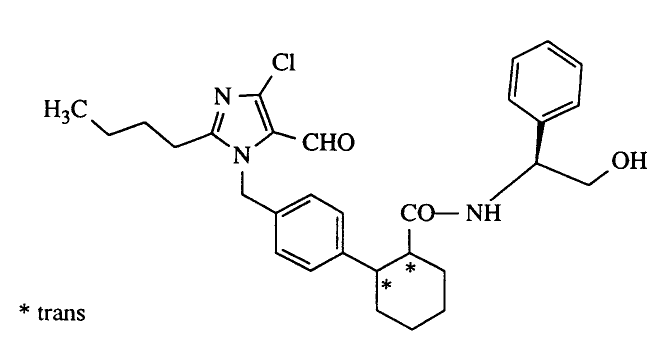

1,3 g (2,8 mmol) der Verbindung aus Beispiel 2 werden bei -30°C in 30 ml Tetrahydrofuran mit 0,78 ml (5,6 mmol) Triethylamin und 0,235 ml (3,1 mmol) Methansulfonsäurechlorid zur Reaktion gebracht. Nach 30 Minuten bei -30°C wird eine Lösung von 459 mg (3,3 mmol) (S)-Phenylglycinol und 0,34 g (2,8 mmol) 4-(N,N-Dimethylamino)pyridin in 10 ml Tetrahydrofuran zugetropft und die Mischung unter Erwärmung auf 20°C 24 h nachgerührt. Man gießt auf 1 M Salzsäure und extrahiert mehrfach mit Ether. Die organischen Phasen werden mit Natriumsulfat getrocknet, eingedampft und der Rückstand chromatographisch aufgetrennt (Kieselgel 60, Merck)
Ausbeute: 186 mg (0,36 mmol) Beispiel 6 (Diastereomer A)
591 mg Beispiel 6/7 (Diastereomerengemisch A+B)
230 mg (0,44 mmol) Beispiel 7 (Diastereomer B)
R_{f} = 0,32 (H) Beispiel 6
R_{f} = 0,17 (H) Beispiel 7

### Beispiel 8

### trans-2-[4-(2-Butyl-4-chlor-5-hydroxymethylimidazol-1-yl-methyl)phenyl]-cyclohexan-1-carbonsäure-tert.butylester

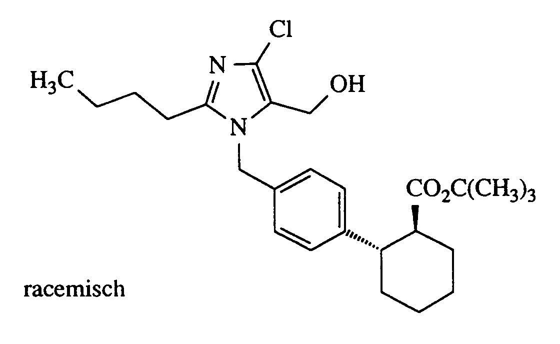

1 g (1,9 mmol) der Verbindung aus Beispiel 1 wird in 10 ml Ethanol gelöst und bei 20°C mit 74,2 mg (2,0 mmol) Natriumboranat umgesetzt. Nach 1 h wird Wasser zugegeben und mit Ether extrahiert. Die organische Phase wird mit Natriumsulfat getrocknet und eingedampft.
Ausbeute: 0,97 g (1,85 mmol)
R_{f} = 0,53 (H)

Analog zu Beispiel 8 werden die in Tabelle 2 aufgeführten Verbindungen hergestellt:

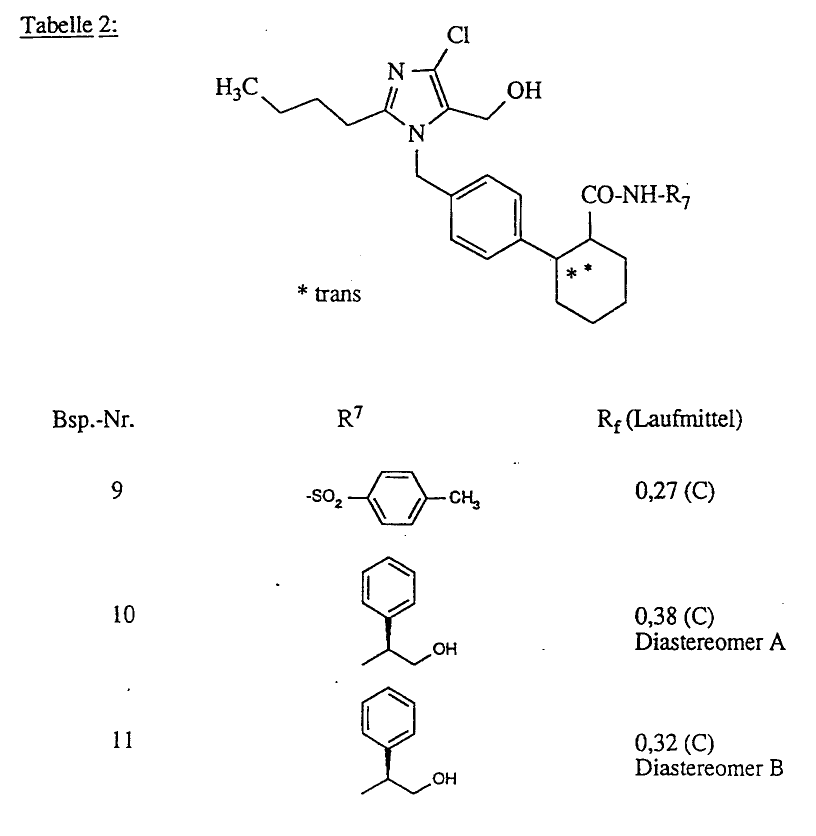

### Beispiel 12

### trans-2-[4-(2-Butyl-4-chlor-5-carboxy-imidazol-1-yl-methyl)phenyl]-cyclohexan-1-carbonsäure-N-(4-tolylsulfonyl)amid

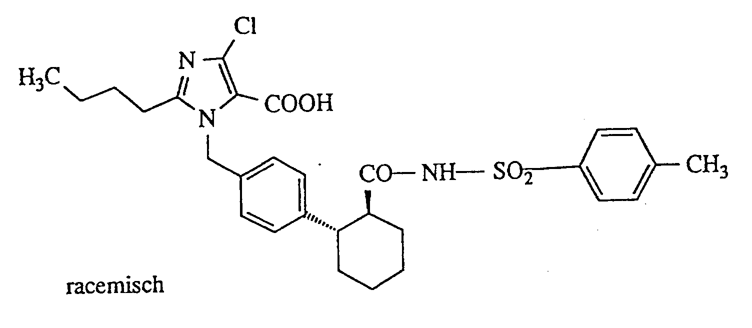

In Analogie zur Vorschrift des Beispiels 12 wird die Titelverbindung hergestellt. R_{f} = 0,11 (C)

## Patentansprüche

1. Imidazolyl-substituierte Cyclohexanderivate der allgemeinen Formel 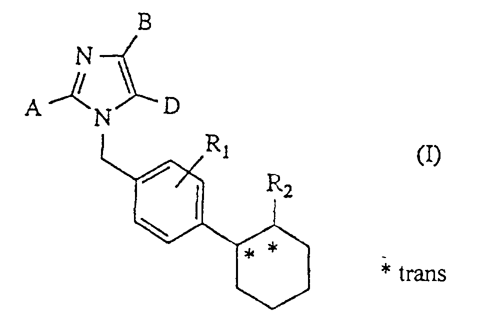 in welcher
A für geradkettiges oder verzweigtes Alkyl oder Alkenyl mit jeweils bis zu 8 Kohlenstoffatomen steht, oder
für Cycloalkyl mit 3 bis 8 Kohlenstoffatomen steht,
B für Wasserstoff, Halogen oder für Perfluoralkyl mit bis zu 5 Kohlenstoffatomen steht,
D für eine Gruppe der Formel-CH₂-OR³ oder -CO-R⁴ steht,
worin
R³ Wasserstoff oder geradkettiges oder verzweigtes Alkyl mit bis zu 8 Kohlenstoffatomen bedeutet,
R⁴ Wasserstoff, Hydroxy oder geradkettiges oder verzweigtes Alkoxy mit bis zu 8 Kohlenstoffatomen bedeutet,
R¹ für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano oder Carboxy steht,
R² für einen Rest der Formel -CO-NR⁶R⁷ steht,
worin
R⁶ Wasserstoff oder geradkettiges oder verzweigtes Alkyl mit bis zu 6 Kohlenstoffatomen bedeutet,
und
R⁷ einen Rest der Formel -SO₂R⁹ oder 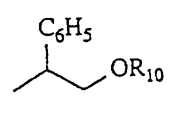 bedeutet,
worin
R⁹ geradkettiges oder verzweigtes Alkyl mit bis zu 8 Kohlenstoffatomen bedeutet, das gegebenenfalls durch Phenyl oder Tolyl substituiert ist, oder
Phenyl bedeutet, das gegebenenfalls durch Halogen oder durch geradkettiges oder verzweigtes Alkyl mit bis zu 6 Kohlenstoffatomen substituiert ist,
R¹⁰ Wasserstoff, geradkettiges oder verzweigtes Alkyl mit bis zu 6 Kohlenstoffatomen oder eine Hydroxyschutzgruppe bedeutet,
und deren Salze.

2. Imidazolyl-substituierte Cyclohexanderivate nach Anspruch 1
wobei
A für geradkettiges oder verzweigtes Alkyl oder Alkenyl mit jeweils bis zu 6 Kohlenstoffatomen steht, oder
für Cyclopropyl, Cyclobutyl, Cyclopentyl, Cyclohexyl oder Cycloheptyl steht,
B für Wasserstoff, Fluor, Chlor, Brom oder für Perfluoralkyl mit bis zu 4 Kohlenstoffatomen steht,
D für eine Gruppe der Formel -CH₂OR³ oder -CO-R⁴ steht,
worin
R³ Wasserstoff oder geradkettiges oder verzweigtes Alkyl mit bis zu 6 Kohlenstoffatomen bedeutet,
R⁴ Wasserstoff, Hydroxy oder geradkettiges oder verzweigtes Alkoxy mit bis zu 6 Kohlenstoffatomen bedeutet,
R¹ für Wasserstoff, Fluor, Chlor, Brom, Trifluormethyl, Carboxy oder geradkettiges oder verzweigtes Alkyl, Alkoxy oder Alkoxycarbonyl mit jeweils bis zu 4 Kohlenstoffatomen steht,
R² für einen Rest der Formel- CO-NR⁶R⁷ steht,
worin
R⁶ Wasserstoff oder geradKettiges oder verzweigtes Alkyl mit bis zu 4 Kohlenstoffatomen bedeutet,
und
R⁷ einen Rest der Formel -SO₂-R⁹ oder 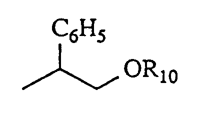 bedeutet,
worin
R⁹ geradkettiges oder verzweigtes Alkyl mit bis zu 6 Kohlenstoffatomen bedeutet, das gegebenenfalls durch Phenyl oder Tolyl substituiert ist, oder
Phenyl bedeutet, das gegebenenfalls durch Fluor, Chlor, Brom, oder durch geradkettiges oder verzweigtes Alkyl mit bis zu 4 Kohlenstoffatomen substituiert ist,
R¹⁰ Wasserstoff oder geradkettiges oder verzweigtes Alkyl mit bis zu 4 Kohlenstoffaotmen oder Benzyl bedeutet,
und deren Salze.

3. Imidazolyl-substituierte Cyclohexanderivate nach Anspruch 1
wobei
A für geradkettiges oder verzweigtes Alkyl oder Alkenyl mit jeweils bis zu 4 Kohlenstoffatomen steht, oder
für Cyclopropyl, Cyclopentyl oder Cyclohexyl steht,
B für Wasserstoff, Fluor, Chlor oder für Perfluoralkyl mit bis zu 3 Kohlenstoffatomen steht,
D für eine Gruppe der Formel -CH₂OR³ oder -CO-R⁴ steht,
worin
R³ Wasserstoff oder geradkettiges oder verzweigtes Alkyl mit bis zu 4 Kohlenstoffatomen bedeutet,
R⁴ Wasserstoff, Hydroxy oder geradkettiges oder verzweigtes Alkoxy mit bis zu 4 Kohlenstoffatomen bedeutet,
R¹ für Wasserstoff, Fluor, Chlor, Brom, Trifluormethyl oder Methyl steht,
R² für einen Rest der Formel -CO-NR⁶R⁷ steht,
worin
R⁶ Wasserstoff, Methyl oder Ethyl bedeutet,
und
R⁷ einen Rest der Formel -SO₂R⁹ oder 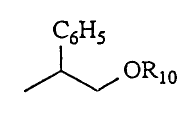 bedeutet,
worin
R⁹ geradkettiges oder verzweigtes Alkyl mit bis zu 4 Kohlenstoffatomen bedeutet, das gegebenenfalls durch Phenyl oder Tolyl substituiert ist, oder Phenyl oder Tolyl bedeutet,
R¹⁰ Wasserstoff, Methyl oder Ethyl bedeutet,
und deren Salze.

4. Imidazolyl-substituierte Cyclohexanderivate nach Anspruch 1 bis 3 zur therapeutischen Anwendung.

5. Verfahren zur Herstellung von Imidazolyl-substituierten Cyclohexan-Derivaten nach Anspruch 1 bis 3 und 9 dadurch gekennzeichnet, daß man Cyclohexan-Verbindungen der allgemeinen Formel 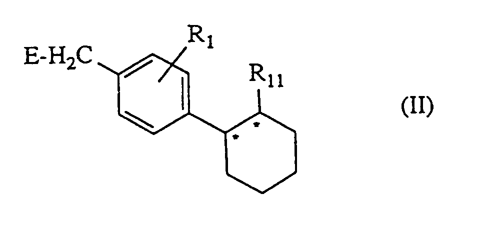 in welcher
E für Chlor, Brom Jod, Tosylat oder Mesylat, vorzugsweise für Brom, steht,
R¹ die oben angegebene Bedeutung hat
und
R¹¹ geradkettiges oder verzweigtes C₁-C₄-Alkoxycarbonyl bedeutet,
zunächst mit Imidazolen der allgemeinen Formel (III) 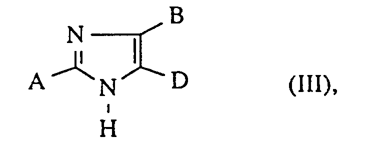 in welcher
A, B und D die oben angegebene Bedeutung haben,
in inerten Lösemitteln, gegebenenfalls in Anwesenheit einer Base und gegebenenfalls unter Schutzgasatmosphäre zu Verbindungen der allgemeinen Formel (IV) 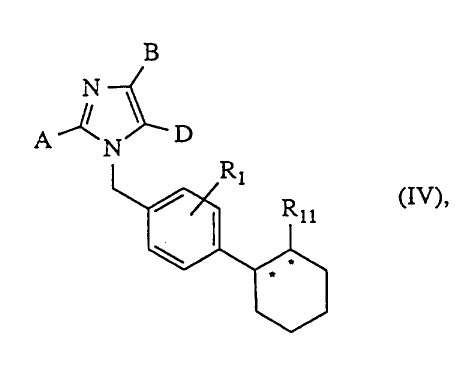 in welcher
A, B, D, R¹ und R¹¹ die oben angegebene Bedeutung haben,
umsetzt,
im Fall der Säuren (R² = CO₂H) die Ester verseift,
und im Fall der Amide und Sulfonamide, ausgehend von den entsprechenden Carbonsäuren, nach vorgeschalteter Aktivierung mit Verbindungen der allgemeinen Formel (V)
H-NR⁶R⁷ (V)
in welcher
R⁶ und R⁷ die oben angegebene Bedeutung haben,
gegebenenfalls in Anwesenheit einer Base und/oder eines Hilfsstoffes, beispielsweise eines Dehydratisierungsmittels, in inerten Lösemitteln amidiert,
und gegebenenfalls die Substituenten A, B, D und R¹ nach üblichen Methoden, beispielsweise durch Reduktion, Oxidation, Alkylierung oder Hydrolyse einführt oder in andere Gruppen überführt,
und gegebenenfalls die Isomeren trennt, und im Fall der Herstellung der Salze mit einer entsprechenden Base oder Säure umsetzt.

6. Arzneimittel enthaltend Imidazolyl-substituierte Cyclohexanderivate nach Anspruch 1 bis 3.

7. Arzneimittel nach Anspruch 6, zur Behandlung von Arteriosklerose

8. Verwendung von Imidazolyl-substituierten Cyclohexanderivaten nach Anspruch 1 bis 3 zur Herstellung von Arzneimitteln.

9. Imidazolyl-substituierte Cyclohexylderivate der allgemeinen Formel 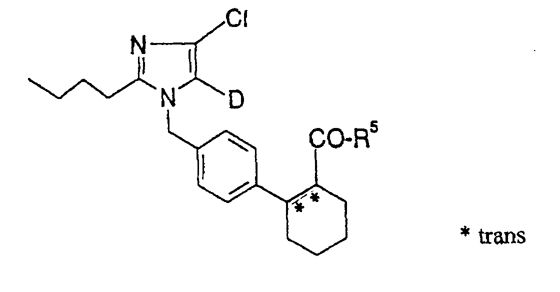 in welcher
entweder D für den Substituenten -CHO steht und R⁵ für Hydroxy oder tert.-Butoxy steht
oder D für die Gruppe -CH₂OH steht und R⁵ für Hydroxy oder tert.-Butoxy steht
oder D für Carboxy und R⁵ für Hydroxy steht und deren Salze.

10. Verwendung von Imidazolyl-substituierten Cyclohexylderivaten nach Anspruch 9 als Zwischenprodukte.

## Claims

1. Imidazolyl-substituted cyclohexane derivatives of the general formula 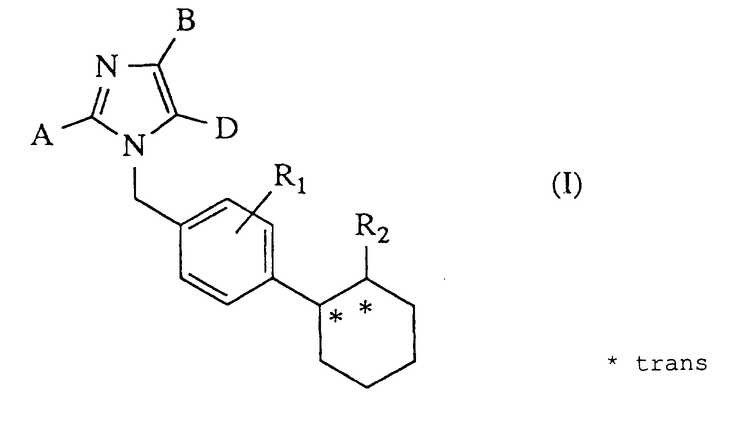 in which
A represents straight-chain or branched alkyl or alkenyl each having up to 8 carbon atoms, or
represents cycloalkyl having 3 to 8 carbon atoms,
B represents hydrogen, halogen or perfluoroalkyl having up to 5 carbon atoms,
D represents a group of the formula -CH₂-OR³ or -CO-R⁴,
in which
R³ denotes hydrogen or straight-chain or branched alkyl having up to 8 carbon atoms,
R⁴ denotes hydrogen, hydroxyl or straight-chain or branched alkoxy having up to 8 carbon atoms,
R¹ represents hydrogen, halogen, nitro, hydroxyl, trifluoromethyl, trifluoromethoxy, straight-chain or branched alkyl, alkoxy or alkoxycarbonyl each having up to 6 carbon atoms, cyano or carboxyl,
R² represents a radical of the formula -CO-NR⁶R⁷,
in which
R⁶ denotes hydrogen or straight-chain or branched alkyl having up to 6 carbon atoms,
and
R⁷ denotes a radical of the formula -SO₂R⁹ or 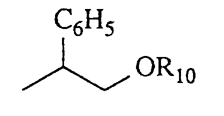 in which
R⁹ denotes straight-chain or branched alkyl having up to 8 carbon atoms, which is optionally substituted by phenyl or tolyl, or
denotes phenyl which is optionally substituted by halogen or by straight-chain or branched alkyl having up to 6 carbon atoms,
R¹⁰ denotes hydrogen, straight-chain or branched alkyl having up to 6 carbon atoms or a hydroxyl protective group,
and their salts.

2. Imidazolyl-substituted cyclohexane derivatives according to Claim 1
where
A represents straight-chain or branched alkyl or alkenyl each having up to 6 carbon atoms, or
represents cyclopropyl, cyclobutyl, cyclopentyl, cyclohexyl or cycloheptyl,
B represents hydrogen, fluorine, chlorine, bromine or perfluoroalkyl having up to 4 carbon atoms,
D represents a group of the formula -CH₂OR³ or -CO-R⁴,
in which
R³ denotes hydrogen or straight-chain or branched alkyl having up to 6 carbon atoms,
R⁴ denotes hydrogen, hydroxyl or straight-chain or branched alkoxy having up to 6 carbon atoms,
R¹ represents hydrogen, fluorine, chlorine, bromine, trifluoromethyl, carboxyl or straight-chain or branched alkyl, alkoxy or alkoxycarbonyl each having up to 4 carbon atoms,
R² represents a radical of the formula -CO-NR⁶R⁷,
in which
R⁶ denotes hydrogen or straight-chain or branched alkyl having up to 4 carbon atoms,
and
R⁷ denotes a radical of the formula -SO₂-R⁹ or 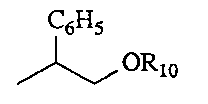 in which
R⁹ denotes straight-chain or branched alkyl having up to 6 carbon atoms, which is optionally substituted by phenyl or tolyl, or
denotes phenyl which is optionally substituted by fluorine, chlorine or bromine, or by straight-chain or branched alkyl having up to 4 carbon atoms,
R¹⁰ denotes hydrogen or straight-chain or branched alkyl having up to 4 carbon atoms or benzyl,
and their salts.

3. Imidazolyl-substituted cyclohexane derivatives according to Claim 1
where
A represents straight-chain or branched alkyl or alkenyl each having up to 4 carbon atoms, or represents cyclopropyl, cyclopentyl or cyclohexyl,
B represents hydrogen, fluorine, chlorine or perfluoroalkyl having up to 3 carbon atoms,
D represents a group of the formula -CH₂OR³ or -CO-R⁴,
in which
R³ denotes hydrogen or straight-chain or branched alkyl having up to 4 carbon atoms,
R⁴ denotes hydrogen, hydroxyl or straight-chain or branched alkoxy having up to 4 carbon atoms,
R¹ represents hydrogen, fluorine, chlorine, bromine, trifluoromethyl or methyl,
R² represents a radical of the formula -CO-NR⁶R⁷,
in which
R⁶ denotes hydrogen, methyl or ethyl,
and
R⁷ denotes a radical of the formula -SO₂R⁹ or 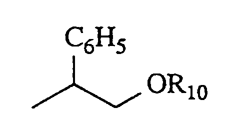 in which
R⁹ denotes straight-chain or branched alkyl having up to 4 carbon atoms, which is optionally substituted by phenyl or tolyl, or denotes phenyl or tolyl,
R¹⁰ denotes hydrogen, methyl or ethyl,
and their salts.

4. Imidazolyl-substituted cyclohexane derivatives according to Claims 1 to 3 for therapeutic use.

5. Process for the preparation of imidazolyl-substituted cyclohexane derivatives according to Claims 1 to 3 and 9, characterised in that cyclohexane compounds of the general formula 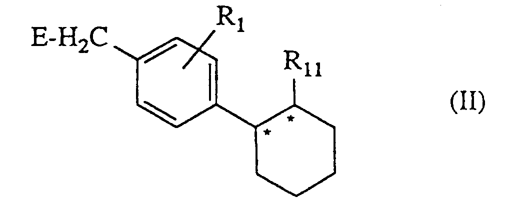 in which
E represents chlorine, bromine, iodine, tosylate or mesylate, preferably bromine,
R¹ has the abovementioned meaning
and
R¹¹ denotes straight-chain or branched C₁-C₄-alkoxycarbonyl,
are reacted first with imidazoles of the general formula (III) 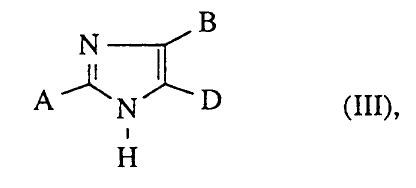 in which
A, B and D have the abovementioned meaning,
in inert solvents, if appropriate in the presence of a base and if appropriate under a protective gas atmosphere, to give compounds of the general formula (IV) 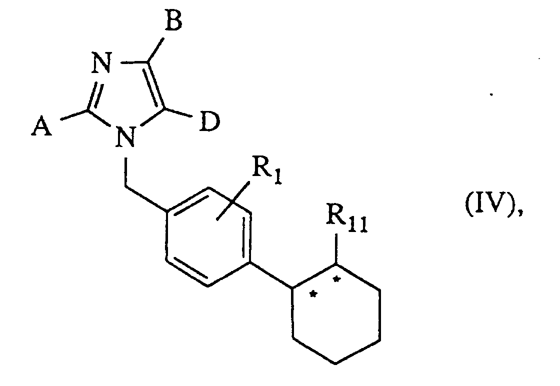 in which
A, B, D, R¹ and R¹¹ have the abovementioned meaning,
in the case of the acids (R² = CO₂H) the esters are hydrolysed,
and in the case of the amides and sulphonamides, starting from the corresponding carboxylic acids, the products are amidated in inert solvents, after prior activation with compounds of the general formula (V)
H-NR⁶R⁷ (V)
in which
R⁶ and R⁷ have the abovementioned meaning,
if appropriate in the presence of a base and/or of an auxiliary, for example of a dehydrating agent,
and if appropriate the substituents A, B, D and R¹ are introduced or converted into other groups by customary methods, for example by reduction, oxidation, alkylation or hydrolysis,
and if appropriate the isomers are separated, and in the case of the preparation of the salts reacted with an appropriate base or acid.

6. Medicaments containing imidazolyl-substituted cyclohexane derivatives according to Claims 1 to 3.

7. Medicaments according to Claim 6, for treating arteriosclerosis.

8. Use of imidazolyl-substituted cyclohexane derivatives according to Claims 1 to 3 for the production of medicaments.

9. Imidazolyl-substituted cyclohexyl derivatives of the general formula 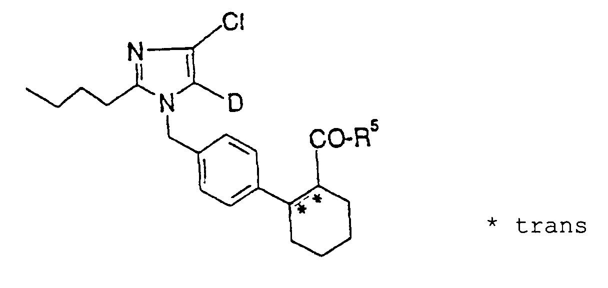 in which
either D represents the substituent -CHO and R⁵ represents hydroxyl or tert-butoxy
or D represents the group -CH₂OH and R⁵ represents hydroxyl or tert-butoxy
or D represents carboxyl and R⁵ represents hydroxyl and their salts.

10. Use of imidazolyl-substituted cyclohexyl derivatives according to Claim 9 as intermediates.

## Revendications

1. Dérivés de cyclohexane à substituant imidazolyle de formule générale 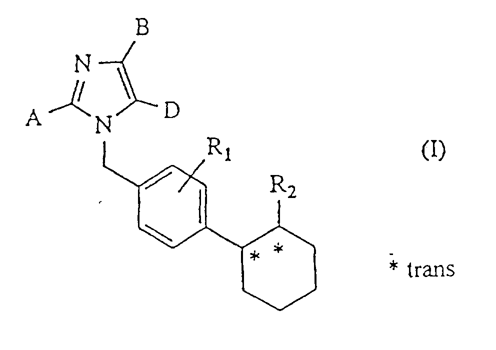 dans laquelle
A est un groupe alkyle ou alcényle linéaire ou ramifié ayant dans chaque cas jusqu'à 8 atomes de carbone, ou bien
un groupe cycloalkyle ayant 3 à 8 atomes de carbone,
B représente l'hydrogène, un halogène ou un groupe perfluoralkyle ayant jusqu'à 5 atomes de carbone,
D est un groupe de formule -CH₂-OR³ ou -CO-R⁴,
dans laquelle
R³ représente l'hydrogène ou un groupe alkyle linéaire ou ramifié ayant jusqu'à 8 atomes de carbone,
R⁴ est l'hydrogène, un groupe hydroxy ou un groupe alkoxy linéaire ou ramifié ayant jusqu'à 8 atomes de carbone,
R¹ représente l'hydrogène, un halogène, un groupe nitro, hydroxy, trifluorométhyle, trifluorométhoxy, un groupe alkyle, alkoxy ou aikoxycarbonyle linéaire ou ramifié ayant dans chaque cas jusqu'à 6 atomes de carbone, un groupe cyano ou carboxy,
R² représente un reste de formule -CO-NR⁶R⁷,
dans laquelle
R⁶ est l'hydrogène ou un groupe alkyle linéaire ou ramifié ayant jusqu'à 6 atomes de carbone,
et
R⁷ est un reste de formule -SO₂R⁹ ou 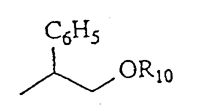 dans laquelle
R⁹ est un groupe alkyle linéaire ou ramifié ayant jusqu'à 8 atomes de carbone, qui est substitué le cas échéant par un radical phényle ou tolyle, ou représente
un groupe phényle qui est substitué le cas échéant par un halogène ou par un radical alkyle linéaire ou ramifié ayant jusqu'à 6 atomes de carbone,
R¹⁰ représente l'hydrogène, un groupe alkyle linéaire ou ramifié ayant jusqu'à 6 atomes de carbone ou un groupe protégeant la fonction hydroxy,
et leurs sels.

2. Dérivés de cyclohexane à substituant imidazolyle suivant la revendication 1,
dans lesquels
A est un groupe alkyle ou alcényle linéaire ou ramifié ayant dans chaque cas jusqu'à 6 atomes de carbone, ou bien
un groupe cyclopropyle, cyclobutyle, cyclopentyle, cyclohexyle ou cycloheptyle,
B représente l'hydrogène, le fluor, le chlore, le brome ou un groupe perfluoralkyle ayant jusqu'à 4 atomes de carbone,
D est un groupe de formule -CH₂-OR³ ou -CO-R⁴,
dans laquelle
R³ est l'hydrogène ou un groupe alkyle linéaire ou ramifié ayant jusqu'à 6 atomes de carbone,
R⁴ représente l'hydrogène, un groupe hydroxy ou un groupe alkoxy linéaire ou ramifié ayant jusqu'à 6 atomes de carbone,
R¹ est l'hydrogène, le fluor, le chlore, le brome, un groupe trifluorométhyle, carboxy ou un groupe alkyle, alkoxy ou alcoxycarbonyle linéaire ou ramifié ayant dans chaque cas jusqu'à 4 atomes de carbone,
R² représente un reste de formule -CO-NR⁶R⁷, dans laquelle
R⁶ est l'hydrogène ou un groupe alkyle linéaire ou ramifié ayant jusqu'à 4 atomes de carbone,
et
R⁷ est un reste de formule -SO₂R⁹ ou 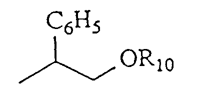 dans laquelle
R⁹ est un groupe alkyle linéaire ou ramifié ayant jusqu'à 6 atomes de carbone, qui est substitué le cas échéant par un radical phényle ou tolyle, ou bien désigne
un groupe phényle qui est substitué le cas échéant par du fluor, du chlore, du brome ou par un radical alkyle linéaire ou ramifié ayant jusqu'à 4 atomes de carbone,
R¹⁰ représente l'hydrogène ou un groupe alkyle linéaire ou ramifié ayant jusqu'à 4 atomes de carbone ou un groupe benzyle
et leurs sels.

3. Dérivés de cyclohexane à substituant imidazolyle suivant la revendication 1, dans lesquels
A est un groupe alkyle ou alcényle linéaire ou ramifié ayant dans chaque cas jusqu'à 4 atomes de carbone ou représente
un groupe cyclopropyle, cyclopentyle ou cyclohexyle,
B est l'hydrogène, le fluor, le chlore ou un groupe perfluoralkyle ayant jusqu'à 3 atomes de carbone,
D représente un groupe de formule -CH₂OR³ ou -CO-R⁴,
dans laquelle
R³ représente l'hydrogène ou un groupe alkyle linéaire ou ramifié ayant jusqu'à 4 atomes de carbone,
R⁴ représente l'hydrogène, un groupe hydroxy ou un groupe alkoxy linéaire ou ramifié ayant jusqu'à 4 atomes de carbone,
R¹ est l'hydrogène, le fluor, le chlore, le brome, un groupe trifluorométhyle, ou méthyle,
R² représente un reste de formule -CO-NR⁶R⁷,
dans laquelle
R⁶ est l'hydrogène, un groupe méthyle ou éthyle,
et
R⁷ représente un reste de formule -SO₂R⁹ ou 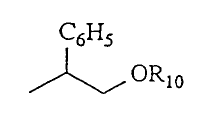 où
R⁹ est un groupe alkyle linéaire ou ramifié ayant jusqu'à 4 atomes de carbone, qui est substitué le cas échéant par un radical phényle ou tolyle, ou représente
un groupe phényle ou tolyle,
R¹⁰ représente l'hydrogène, un groupe méthyle ou éthyle,
et leurs sels.

4. Dérivés de cyclohexane à substituant imidazolyle suivant les revendications 1 à 3, destinés à une application thérapeutique.

5. Procédé de production de dérivés de cyclohexane à substituant imidazolyle suivant les revendications 1 à 3 et 9, caractérisé en ce qu'on fait réagir des composés de cyclohexane de formule générale 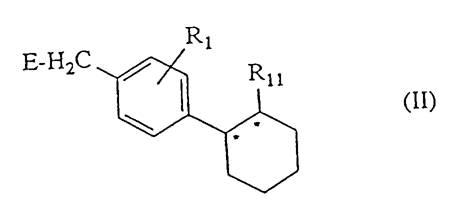 dans laquelle
E Représente le chlore, le brome, l'iode, le groupe tosylate ou mésylate, de préférence le brome,
R¹ a la définition indiquée ci-dessus,
et
R¹¹ est un groupe (alkoxy en C₁ à C₄)carbonyle linéaire ou ramifié,
tout d'abord avec des imidazoles de formule générale (III) 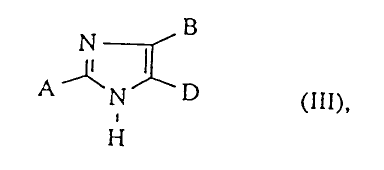 dans laquelle
A, B et D ont les définitions indiquées ci-dessus,
dans des solvants inertes, le cas échéant en présence d'une base et éventuellement sous atmosphère de gaz protecteur pour obtenir des composés de formule générale (IV) 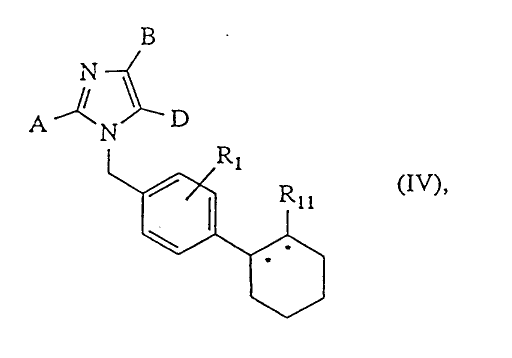 dans laquelle
A, B, D, R¹ et R¹¹ ont la définition indiquée ci-dessus,
on saponifie les esters dans le cas des acides (R²=CO₂H),
et dans le cas des amides et sulfonamides, en partant des acides carboxyliques correspondants, on effectue une amidation après activation préalable avec des composés de formule générale (V)
H-NR⁶R⁷ (V)
dans laquelle
R⁶ et R⁷ ont la définition indiquée ci-dessus,
le cas échéant en présence d'une base et/ou d'une substance auxiliaire, par exemple un agent de déshydratation, dans des solvants inertes,
et le cas échéant, on introduit les substituants A, B, D et R¹ selon des modes opératoires classiques, par exemple par réduction, oxydation, alkylation ou hydrolyse ou on les transforme en d'autres groupes,
et on sépare éventuellement les isomères, et dans le cas de la préparation des sels, on conduit la réaction avec une base ou un acide correspondant.

6. Médicaments contenant des dérivés de cyclohexane à substituant imidazolyle suivant les revendications 1 à 3.

7. Médicament suivant la revendication 6, destiné au traitement de l'artériosclérose.

8. Utilisation de dérivés de cyclohexane a substituant imidazolyle suivant les revendications 1 à 3 pour la préparation de médicaments.

9. Dérivés cyclohexyliques à substituant imidazolyle de formule générale 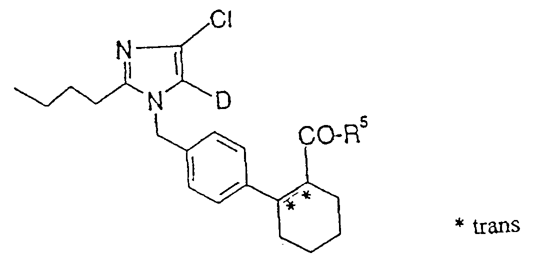 dans laquelle
D représente le substituant -CHO et R⁵ est un groupe hydroxy ou tertio-butoxy,
ou bien D représente le groupe -CH₂OH et R⁶ est un groupe hydroxy ou tertio-butoxy,
ou bien D est un groupe carboxy et R⁵ est un groupe hydroxy,
et leur sels.

10. Utilisation de dérivés cyclohexyliques à substituant imidazolyle suivant la revendication 9 comme produits intermédiaires.
